(19) Europäisches Patentamt
European Patent Office
Office européen des brevets

(11) EP 2 835 370 A1

(12) # EUROPEAN PATENT APPLICATION

(43) Date of publication:
**11.02.2015 Bulletin 2015/07**

(21) Application number: **13179693.0**

(22) Date of filing: **08.08.2013**

(51) Int Cl.:
***C07D 401/12*** *(2006.01)* ***A61K 31/4439*** *(2006.01)*
***A61P 7/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Medichem, S.A.**
**08970 Barcelona (ES)**

(72) Inventors:
- **Gavaldá Escudé, Ana**
  **E-08173 Sant Cugat del Vallès (ES)**
- **Mohnani, Stefan**
  **Birkirkara, BKR4243 (MT)**
- **Durán López, Ernesto**
  **E-08755 Castellbisbal (ES)**

(74) Representative: **ABG Patentes, S.L.**
**Avenida de Burgos, 16D**
**Edificio Euromor**
**28036 Madrid (ES)**

(54) **New crystals of dabigatran etexilate mesylate**

(57) The invention relates to a carbamimidoylphenylaminomethylbenzimidazole derivative with modified specific surface area and optionally particle size distribution compared to the compound known from the prior art.

EP 2 835 370 A1

## Description

BACKGROUND OF THE INVENTION

The present invention is related to dabigatran etexilate mesylate, processes of preparing dabigatran etexilate mesylate, and pharmaceutical compositions and therapeutic uses thereof. In particular, the present invention relates to a dabigatran etexilate mesylate with modified specific surface area and optionally particle size distribution compared to the dabigatran etexilate mesylate known from the prior art.

Dabigatran etexilate, compound of formula (I), is the international commonly accepted non-proprietary name for ethyl 3-{[(2-{[(4-{[(hexyloxy)carbonyl]-carbamimidoyl}phenyl)amino]methyl}-1-methyl-1*H*-benzimidazol-5-yl)carbonyl]-(pyridin-2-yl)amino}propanoate, which has an empirical formula of $C_{34}H_{41}N_7O_5$ and a molecular weight of 627.73.

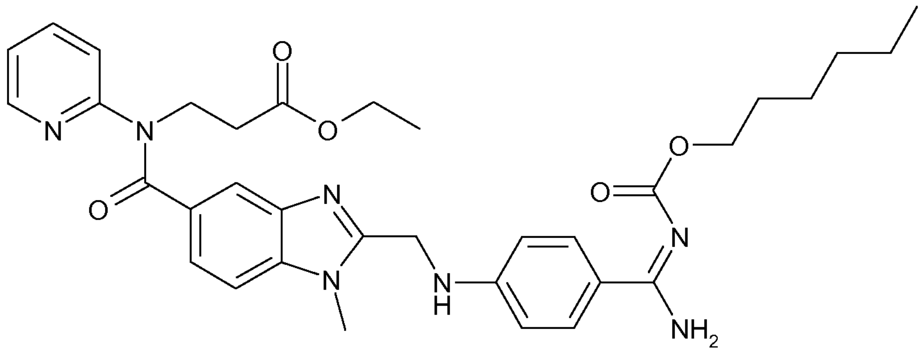

**(I)**

Dabigatran etexilate is the pro-drug of the active substance, dabigatran, which has a molecular formula $C_{25}H_{25}N_7O_3$ and molecular mass 471.51. The mesylate salt (1:1) of dabigatran etexilate is known to be therapeutically useful one and is commercially marketed as oral hard capsules in the United States and in Europe under the trade mark Pradaxa™ for the prevention of stroke and systemic embolism in patients with non-valvular atrial fibrillation. Additionally, it is also marketed in Europe under the same trade mark for the primary prevention of venous thromboembolic events in adult patients who have undergone elective total hip replacement surgery or total knee replacement surgery.

Dabigatran etexilate mesylate was first described in European Patent No. EP 1.870.100 B1. Concretely, Example 3 of this patent discloses the precipitation of the mesylate salt by stirring a solution of Dabigatran etexilate in ethyl acetate, in the presence of one molar equivalent of methanesulfonic acid, at room temperature. PCT Patent Application No. WO 2011/110876 A1 also discloses the precipitation of Dabigatran etexilate mesylate from ethyl acetate.

Most of the prior art processes for the preparation of dabigatran etexilate mesylate disclose its precipitation from acetone solvent, or from a mixture of solvents comprising acetone. For example, United States patents No. US 7,202,368 B2, US 7,932,273 B2, US 8,354,543 B2, US 8,394,962 B2 and US 8,399,678 B2, as well as PCT patent applications No. WO 2012/077136 A2, WO 2012/152855 A1 and WO 2013/024384 A1 disclose the precipitation of dabigatran etexilate mesylate from acetone. On the other hand, United States patent No. US 7,880,016 B2 discloses the precipitation of dabigatran etexilate mesylate from a mixture of butyl acetate, acetone and ethanol; United States patent No. US 8,354,543 B2 discloses its precipitation from a mixture of methyl isobutyl ketone and acetone; PCT patent application No. WO 2012/077136 A2 discloses its precipitation from a mixture of acetone and methanol; and PCT patent application No. WO 2012/152855 A1 discloses its precipitation from a mixture of dichloromethane and acetone.

The precipitation of dabigatran etexilate mesylate from other solvents or mixtures of solvents not comprising ethyl acetate or acetone is disclosed in just a few prior art documents. Concretely, PCT patent application No. WO 2012/027543 A1 discloses the precipitation of dabigatran etexilate mesylate from cyclohexanone, 1-pentanol, tetrahydrofuran, ethyl acetoacetate, 2-methoxyethyl ether and 2-methyltetrahydrofuran solvents. Similarly, PCT patent application No. WO 2012/152855 A1 discloses its preparation from tetrahydrofuran. Additionally, PCT patent application No. WO 2012/077136 A2 discloses its precipitation from a mixture of methyl isobutyl ketone and methanol.

A problem associated with the use of dabigatran etexilate mesylate is that it is slightly insoluble in pure water (1.8 mg/mL). The low solubility is particularly problematic with respect to an oral application, which results in a poor bioavailability.

The drug absorption rate of poorly water-soluble drugs can be particularly affected by the specific surface area because, for these drugs, the bioavailability is dissolution-rate controlled in most cases.

None of the above prior art references disclose the specific surface area properties of the isolated dabigatran

etexilate mesylate.

PCT patent application No. WO 2012/077136 A2 discloses that dabigatran etexilate mesylate can be further micronized or milled to get the desired particle size. It also discloses that the techniques that may be used for particle size reduction include, without limitation, ball, roller and hammer mills, and jet mills. Additionally, it discloses that milling or micronization may be performed before drying, or after the completion of drying of the product. However, this patent application does not give any detail related to the equipment and operating conditions used for milling or micronizing dabigatran etexilate mesylate, and does not give any details about the physical characteristics of the dabigatran etexilate mesylate obtained before the milling or micronizing step. It is of common knowledge that the properties of a milled or micronized material could be strongly affected by the specific properties of the equipment and the specific procedure used for the milling or micronizing step.

PCT patent application No. WO 2012/001156 A2 discloses that the dabigatran etexilate mesylate used in the preparation of pharmaceutical compositions preferably has a $D_{90}$ value of less than 150 $\mu$m, more preferably less than 80 $\mu$m, and most preferably less than 40 $\mu$m. Concretely, Example C2 of this patent application describes the preparation of pellets containing dabigatran etexilate mesylate, by addition of milled dabigatran etexilate mesylate having a $D_{90}$ value of 15.7 $\mu$m and a $D_{50}$ value of 6.7 $\mu$m. However, the patent application does not disclose any procedure for the preparation of the milled dabigatran etexilate mesylate having these specific particle size properties, or the technique used therein for measuring the particle size distribution.

Similarly, PCT patent application No. WO 2011/110478 A1 discloses that dabigatran etexilate or a pharmaceutically acceptable salt thereof is present in a pharmaceutical composition having a $D_{90}$ value of less than 50 $\mu$m, preferably less than 30 $\mu$m, and most preferably of less than 10 $\mu$m. However, the patent application does not disclose any procedure for the preparation of dabigatran etexilate mesylate having these specific particle size properties. Moreover, the experimental examples of this patent application disclose the dissolution of the dabigatran etexilate mesylate during the formulation procedure (Examples 1 to 8) or the grinding of a physical mixture of dabigatran etexilate mesylate in the presence of the corresponding pharmaceutical excipients (Examples 9 to 11). Consequently, the particle size properties of the dabigatran etexilate mesylate are expected to be substantially modified during such formulation processes and, therefore, the particle size properties of the active pharmaceutical ingredient used as starting material for the disclosed formulation experiments might be substantially different from the above preferred $D_{90}$ values. Additionally, the patent application does not disclose the technique used therein for measuring the particle size distribution.

None of the above references provide the specific surface area of the milled or micronized dabigatran etexilate mesylate. In this regard, it is established and well understood in the field of particle size measurement that particle size and/or particle size distribution cannot be directly related to the specific surface area of particles, since any assumption about the particle shape and as such specific surface area fails to account for specific surface texture and/or specific surface contours and/or specific surface defects and/or localized surface properties of the particles, any and all of which can have an influence on specific surface area and associated properties.

Moreover, the surface properties and in particular specific surface area can be strongly affected, for example, by the energy input associated with the process used for the reduction of particle size. Therefore depending on the process used for reducing the particle size, it is possible to obtain different specific surface areas for particles having similar particle size distribution.

Additionally, it is of general knowledge that the physical properties of the starting material before milling or micronizing might strongly affect the properties, in terms of particle size distribution as well as in terms of specific surface area, of the final micronized material. For example, it is well known that properties of milled solids can be influenced by the morphology of the starting material used for the milling (Eur. J. Pharm. Sci. 2006, 27, 19-26). None of the references in the prior art disclose properly the properties and/or the preparation procedures of the dabigatran etexilate mesylate used as a starting material for the milling or micronization processes.

Based on the prior art, there is a need for dabigatran etexilate mesylate with improved properties for pharmaceutical formulation and such dabigatran etexilate mesylate is now provided by the present invention substantially as hereinafter described in greater detail.

## BRIEF DESCRIPTION OF THE DRAWINGS

**Figure 1** depicts the X-Ray Powder Diffraction (XRPD) plot of Form I of dabigatran etexilate mesylate.

**Figure 2** depicts the Differential Scanning Calorimetry (DSC) plot of Form I of dabigatran etexilate mesylate, substantially free of Form II.

## BRIEF SUMMARY OF THE INVENTION

The invention relates to dabigatran etexilate mesylate crystals having a high specific surface area, to processes for the preparation thereof, and to the use thereof for preparing pharmaceutical compositions.

In particular, the invention related to dabigatran etexilate mesylate crystals having a high specific surface area and an increased solubility, and to processes for the preparation thereof.

DETAILED DESCRIPTION OF THE INVENTION

There is now provided by the present invention dabigatran etexilate mesylate with improved properties.

In one aspect, the dabigatran etexilate mesylate of the present invention can be characterized and distinguished from the prior art by reference to its specific surface area. According to the present invention, there is provided dabigatran etexilate mesylate characterized by a specific surface area in the range of from about 7.0 $m^2/g$ to about 20 $m^2/g$, preferably from about 7.5 $m^2/g$ to about 15 $m^2/g$, and even more preferably from about 8.0 $m^2/g$ to about 13 $m^2/g$. The specific surface area of a material is defined as the total surface area of this material divided by its mass. Suitable methods to measure the specific surface area of a material are common knowledge in the art, examples are given below.

Preferably dabigatran etexilate mesylate as provided by the present invention is mechanically comminuted. Comminution is the process in which solid materials are reduced in size. In one embodiment of the present invention, dabigatran etexilate mesylate is obtained by a mechanical size reduction (*i.e.,* mechanical comminution) process, preferably by cutting, chipping, grinding, crushing, milling, micronizing, triturating and the like. In a preferred embodiment, the present invention provides micronized or milled dabigatran etexilate mesylate suitable for pharmaceutical use.

The known dabigatran etexilate mesylate which is obtained by the synthetic processes disclosed in the prior art, which consequently do not involve a mechanical comminution step, has a specific surface area substantially below 7.0 $m^2/g$. Particularly, the dabigatran etexilate mesylate obtained by crystallization from a solvent comprising acetone, which is the most frequent solvent disclosed in the prior art processes, has a specific surface area of about 3 $m^2/g$.

Advantageously, dabigatran etexilate mesylate according to the present invention preferably has a $D_{90}$ particle size value of less than about 25 μm, preferably less than about 22 μm, and even more preferred less than about 20 μm.

In a preferred embodiment, the dabigatran etexilate mesylate according to the present invention is further characterized by having a $D_{50}$ particle size value of less than about 15 μm, preferably less than about 12 μm, and even more preferred less than about 10 μm.

In another preferred embodiment, the dabigatran etexilate mesylate according to the present invention is further characterized by having a $D_{10}$ particle size value of less than about 5 μm, preferably less than about 3 μm, and even more preferred less than about 2 μm.

In particular, the dabigatran etexilate mesylate according to the present invention is characterized by a specific surface area in the range of from about 7.0 $m^2/g$ to about 20 $m^2/g$, preferably from about 7.5 $m^2/g$ to about 15 $m^2/g$, and even more preferred from about 8.0 $m^2/g$ to about 13 $m^2/g$, and a $D_{90}$ particle size value of less than about 25 μm, preferably less than about 22 μm, and even more preferred less than about 20 μm.

In a preferred embodiment, the dabigatran etexilate mesylate according to the present invention is characterized by a specific surface area in the range of from about 7.0 $m^2/g$ to about 20 $m^2/g$, preferably from about 7.5 $m^2/g$ to about 15 $m^2/g$, and even more preferred from about 8.0 $m^2/g$ to about 13 $m^2/g$, a $D_{90}$ particle size value of less than about 25 μm, preferably less than about 22 μm, and even more preferred less than about 20 μm, and a $D_{50}$ particle size value of less than about 15 μm, preferably less than about 12 μm, and even more preferred less than about 10 μm.

In a further preferred embodiment, the dabigatran etexilate mesylate according to the present invention is characterized by a specific surface area in the range of from about 7.0 $m^2/g$ to about 20 $m^2/g$, preferably from about 7.5 $m^2/g$ to about 15 $m^2/g$, and even more preferred from about 8.0 $m^2/g$ to about 13 $m^2/g$, a $D_{90}$ particle size value of less than about 25 μm, preferably less than about 22 μm, and even more preferred less than about 20 μm, a $D_{50}$ particle size value of less than about 15 μm, preferably less than about 12 μm, and even more preferred less than about 10 μm, and a $D_{10}$ particle size value of less than about 5 μm, preferably less than about 3 μm, and even more preferred less than about 2 μm.

The notation $D_x$ [also written as D(v, 0.X)] means that X % of the particles (volume distribution) have a diameter value below a specified diameter D. Thus a $D_{90}$ [or D(v, 0.9)] of 25 μm means that 90% of the particles (volume distribution) have a diameter of less than 25 μm. Similarly, a $D_{50}$ [or D(v, 0.5)] of 15 μm means that 50% of the particles (volume distribution) have a diameter of less than 15 μm. Also, a $D_{10}$ [or D(v, 0.1)] of 5 μm means that 10% of the particles (volume distribution) have a diameter of less than 5 μm. Suitable methods to measure the Dx values of a material are common knowledge in the art, examples are provided below.

The dabigatran etexilate mesylate according to the present invention preferably corresponds to crystalline form I of dabigatran etexilate mesylate. The crystalline form I of dabigatran etexilate mesylate is characterized by a melting point of 180 °C ± 3 °C as determined by peak maximum evaluation of a Differential Scanning Calorimetry (DSC) plot at a heating rate of 10°C/min. The crystalline form I of dabigatran etexilate mesylate is further characterized by having a X-ray Powder Diffraction (XRPD) pattern which comprises at least peaks (2-theta, 2θ) at 4.4, 11.0 and 18.1° (± 0.2 degrees), preferably at 4.4, 8.9, 11.0, 13.5, 17.7, 18.1, 19.9, 22.1 and 22.9° (± 0.2 degrees), more preferably at 4.4, 8.9, 9.2, 9.6, 10.6, 11.0, 12.7, 13.5, 14.0, 14.3, 15.2, 15.9, 16.5, 17.7, 18.1, 18.6, 19.9, 20.3, 20.5, 21.1, 22.1, 22.9, 24.1,

25.1, 26.0, 26.5, 26.8, 27.2, 27.6, 28.1, 29.1, 29.3 and 30.0 ° (± 0.2 degrees).

Preferably, the dabigatran etexilate mesylate according to the present invention relates to substantially pure crystalline form I of dabigatran etexilate mesylate. This pure crystalline form does not comprise any other crystalline or amorphous forms, solvates, hydrates, etc.

The particle density ($\rho_{particle}$) of a crystal is a constant value for a specific crystalline form. The true density of a crystal refers to mass of solid material divided by its exact volume without porosity, and it can be directly calculated based on the crystal structure of the compound, as determined by X-Ray crystallography. However, this procedure is not common for such determination, as single crystal X-Ray diffraction is not a routine technique. In this sense, it is also understood that the density of a solid can be experimentally measured using a pycnometer, preferably a helium pycnometer, if the crystal structure is not available. The helium pycnometer is considered to give the closest approximation to the true density, since helium penetrates into the smallest pores and crevices, and permits to approach the real volume. In this regard, according to an advantageous embodiment, the dabigatran etexilate mesylate according to the present invention can suitably be further characterized by a measured experimental particle density in the range of from about 1.200 g/cm$^3$ to about 1.400 g/cm$^3$, preferably a measured experimental particle density in the range of from about 1.250 g/cm$^3$ to about 1.350 g/cm$^3$, and even more preferred a measured experimental particle density of from about 1.300 g/cm$^3$ to about 1.320 g/cm$^3$, which values are measured by helium pycnometry. As is recognized in the art, values for such measured experimental particle density are likely to have some variation from an exact true density value based on crystal structure.

In a further preferred embodiment, the dabigatran etexilate mesylate of the present invention is substantially free of crystalline form II. The crystalline form II of dabigatran etexilate mesylate is characterized by a melting point of 190 °C ± 3 °C as determined by peak maximum evaluation of a Differential Scanning Calorimetry (DSC) plot at a heating rate of 10°C/min. Concretely, the dabigatran etexilate mesylate of the present invention does not show any detectable endothermic peak between 185.0°C and 195.0°C as measured by Differential Scanning Calorimetry (DSC) at a heating rate of 10°C/min. The concept "detectable endothermic peak" as used herein is thus intended to denote an endothermic peak having a normalized integral value of not more than 5.0 J/g (J per g of dabigatran etexilate mesylate), preferably not more than 3.0 J/g, and even more preferably not more than 1.0 J/g. Since the endset temperature of the melting peak corresponding to crystalline form I is usually within the range between 185.0°C and 195.0°C, the presence of a secondary endothermic peak corresponding to crystalline form II is preferably detected by analyzing the second derivative of the original DSC plot, since any inflection of the original curve is easily identified as a separate peak in the second derivative curve.

The bulk density ($\rho_{bulk}$) of a powder is the ratio of the mass of an untapped powder sample and its volume including the contribution of the interparticulate void volume. Hence, the bulk density depends on both the density of powder particles and the spatial arrangement of particles in the powder bed.

Porosity (Φ) of a material is a measure of the void spaces in the material, and is a fraction of the volume of voids over the total volume. Porosity is represented by a value between 0 and 1. The porosity value of a specific material can be calculated from the bulk density ($\rho_{bulk}$) and the particle density ($\rho_{particle}$) of this material:

$$\phi = 1 - \frac{\rho_{bulk}}{\rho_{particle}}$$

Drug substances having a lower porosity value are usually preferred for formulation processes since the bulk density of these substances is increased. For example, larger capsules are usually required for compounds having low bulk densities.

In a preferred embodiment, the dabigatran etexilate mesylate of the present invention has a bulk density of at least about 0.150 g/cm$^3$.

In a further preferred embodiment, the dabigatran etexilate mesylate of the present invention has a porosity not higher than about 0.90.

In a preferred embodiment, the dabigatran etexilate mesylate of the present invention as hereinbefore described is obtained by a process comprising:

i) providing a solution of dabigatran etexilate in an ether solvent;

ii) combining the solution of step (i) with methanesulfonic acid;

iii) optionally seeding with dabigatran etexilate mesylate in crystalline form I;

iv) removing the solvent to obtain dabigatran etexilate mesylate; and

v) optionally mechanical comminution.

It is well known that properties of milled solids can be influenced by the properties (such as morphology) of the starting material used for the milling (Eur. J. Pharm. Sci. 2006, 27, 19-26). However, it is not possible to predict the properties of the milled solids from the properties of the starting material used for the milling.

In this sense, the authors of the present invention have found that a dabigatran etexilate mesylate having a surprisingly high specific surface area is obtained when applying a mechanical comminution process to a starting dabigatran etexilate mesylate obtained by the hereinbefore described process according to the present invention. Particularly, despite the fact that the particle size properties (concretely, the $D_{90}$, $D_{50}$ and $D_{10}$ values) for the dabigatran etexilate mesylate as crystallized from a solvent comprising an ether solvent as hereinbefore described, and those for the dabigatran etexilate mesylate as crystallized from a solvent comprising the most typical solvent disclosed in the prior art procedures (*i.e.,* acetone) are substantially equivalent, the specific surface area of the dabigatran etexilate mesylate as obtained after applying exactly the same mechanical comminution process is substantially higher for the dabigatran etexilate mesylate crystals that have been obtained by mechanical comminution of a dabigatran etexilate mesylate crystallized from a solvent comprising an ether solvent as hereinbefore described. Concretely, the specific surface area of the dabigatran etexilate mesylate as obtained after applying a mechanical comminution process to a dabigatran etexilate mesylate crystallized from a solvent comprising an ether solvent as hereinbefore described is always higher than 7.0 $m^2/g$, and sometimes even higher than 10.0 $m^2/g$, while the specific surface area of the dabigatran etexilate mesylate as obtained after applying exactly the same mechanical comminution process to a dabigatran etexilate mesylate crystallized from a solvent comprising acetone is always below 7.0 $m^2/g$, and sometimes even lower than 6.0 $m^2/g$. After removing the solvent to obtain dabigatran etexilate mesylate a further step of mechanical comminution can optionally be carried out in order to provide for the preferred $D_{90}$, $D_{50}$, and/or $D_{10}$ as defined above.

The dabigatran etexilate mesylate according to the present invention shows a higher dissolution rate in aqueous environments.

Increasing the specific surface area of a particle is usually related with an increase of its porosity. The high energy input associated with a mechanical comminution process usually affects the surface properties, and consequently the bulk properties, of the resulting product. The high energy input can cause a disruption of the crystal lattice on the particle surface and the creation of defects *(*Pharm. Dev. Technol. 2004, 9, 1-13), which is consequently related with an increase of the porosity. However, the authors of the present invention have unexpectedly found that, despite its higher specific surface area, the dabigatran etexilate mesylate of the present invention shows similar porosity values than those observed for the dabigatran etexilate mesylate having a lower specific surface area. Similarly, it has been unexpectedly found that the dabigatran etexilate mesylate of the present invention shows similar bulk density values than those observed for the dabigatran etexilate mesylate having a lower specific surface area.

In a further preferred embodiment, the ether solvent is selected from a group comprising 1,2-dimethoxyethane, 1,4-dioxane, tetrahydrofuran, 2-methyltetrahydrofuran, anisole, tert-butyl methyl ether, diethyl ether, diisopropyl ether, diphenyl ether, cyclopentyl methyl ether, 1,1-diethoxypropane, 1,1-dimethoxypropane, 2,2-dimethoxypropane and mixtures thereof, preferably from a group comprising tetrahydrofuran, 2-methyltetrahydrofuran and mixtures thereof, more preferably comprising tetrahydrofuran.

The present invention still further provides a process for preparing dabigatran etexilate mesylate as hereinbefore described and there is thus provided a process comprising:

i) providing dabigatran etexilate mesylate; and

ii) reducing the particle size of the dabigatran etexilate mesylate of step (i) with a method selected from the group consisting of cutting, chipping, grinding, crushing, milling, micronizing and triturating.

In a preferred embodiment of the present invention, said process comprises:

a) milling dabigatran etexilate mesylate;

b) sieving the milled crystals through a screen having a diameter of 1000 μm or less;

c) separating the sieved crystals; and

d) optionally repeating steps (a) to (c).

Preferably, the method for reducing the particle size of the dabigatran etexilate mesylate of step (ii) as hereinbefore described comprises air-jet milling or pin milling.

The present invention still further provides dabigatran etexilate mesylate obtained by a process substantially as hereinbefore described.

There is also provided by the present invention a pharmaceutical composition comprising an effective amount of dabigatran etexilate mesylate according to the present invention as hereinbefore described, together with a pharmaceutically acceptable carrier, diluent or excipient therefor. The term "effective amount" as used herein means an amount of dabigatran etexilate mesylate that is capable of preventing and/or treating thromboembolic diseases. By "pharmaceutically acceptable carrier, diluent or excipient" is meant that the carrier, diluent or excipient must be pharmaceutically inactive and compatible with dabigatran etexilate mesylate and not be deleterious to a recipient thereof. Suitable pharmaceutically acceptable compositions according to the present invention are preferably solid compositions, such as powder or lyophilized product for inclusion in a suspension or dispersion for an injectable composition, or powder for an oral suspension, tablets, coated tablets such as film coated tablets, non-coated tablets, orodispersible tablets, pellets, pills, granules, capsules, or mini-tablets in capsules; or liquid compositions, such as liquid parenteral or oral compositions in the form of a solution.

The present invention still further provides a pharmaceutical composition comprising dabigatran etexilate mesylate and one or more pharmaceutically acceptable carriers, diluents or excipients wherein said dabigatran etexilate mesylate is formed from dabigatran etexilate mesylate according to the present invention as hereinbefore described. "Formed" means that said dabigatran etexilate mesylate according to the present invention as described above is used to produce the pharmaceutical composition.

Another aspect of the present invention is a process for producing a pharmaceutical composition wherein dabigatran etexilate mesylate according to the present invention as hereinbefore described is admixed with one or more pharmaceutically acceptable carriers, diluents or excipients.

The present invention further provides dabigatran etexilate mesylate according to the present invention, for use in the prevention and/or treatment of thromboembolic diseases or for the manufacture of a medicament for the prevention and/or treatment of thromboembolic diseases. For example, it can be used to reduce the risk of stroke and systemic embolism in patients with non-valvular atrial fibrillation. Preferably, it is used to prevent the formation of blood clots in the veins in adults who have had an operation to replace a hip or knee. It may also be used to prevent stroke and the formation of clots in adults who have an abnormal heart beat called 'non-valvular atrial fibrillation' and are considered to be at risk of stroke.

The present invention also provides a method of prevention and/or treatment of thromboembolic diseases, which method comprises administering to the patient an effective amount of dabigatran etexilate mesylate according to the present invention or a pharmaceutical composition according to the present invention.

The particle size parameters measured in the present invention can for example be obtained by means of a laser light diffraction technique, and specifically by means of a Malvern Mastersizer S particle size analyzer having characteristics as set out below. Namely, the laser source used was a 2 milliwatt helium/neon laser (633 nm wavelength); the detection system was a Fourier Transform lens system; the sample was run using a 2.40 mm lens; the sample unit was a sample unit for wet measurement, and particularly was a MS1 small volume sample dispersion unit stirred cell. The wet dispersion was prepared by using Isopar™ G as a dispersant, and the dispersion was controlled by stirring the unit cell. Regarding the analyzed samples, these were prepared by wetting a weighed amount of dabigatran etexilate mesylate (approximately 60 mg) with 20 mL of a 5% solution (w/v) of soybean lecithin in Isopar™ G, which was prepared by dissolving 2.5 g of soybean lecithin in 50 mL of Isopar™ G; sonicating the sample for 3 minutes, delivering the sample dropwise to the previously background and corrected measuring cell filled with dispersant (Isopar™ G) until the obscuration reached the desired level (13-18%). The characterization parameters, based on volume distributions, were measured for at least six times for each sample, and the result is a mean of said measured values for each sample. Alternatively, a Malvern Mastersizer 2000 equipment with a Hydro 2000S small volume sample dispersion unit stirred cell was also used for the particle size measurement of dabigatran etexilate mesylate. Samples for analysis with the Malvern Mastersizer 2000 equipment were prepared by wetting a weighed amount of dabigatran etexilate mesylate (approximately 60 mg) with 20 mL of a 0.75% solution (w/v) of soybean lecithin in Isopar™ G, which was prepared by dissolving 1.5 g of soybean lecithin in 200 mL of Isopar™ G. Other parameters were not changed with respect to those disclosed in the above method for the Malvern Mastersizer S equipment, except for the background measurement time, which was set to 2 s. Methods were checked to be fully equivalent by performing a comparative analysis of a defined set of samples by using both particle size analysis methods.

The specific surface area values disclosed in the present invention can for example be obtained by means of a specific surface area analysis technique based on the BET (Brunauer, Emmett and Teller) theory, which is a well-accepted theory known in the art for the calculation of surface areas of solids by means of measuring their physical adsorption of gas molecules (see S. Brunauer, P.H. Emmett and E. Teller, J. Am. Chem. Soc. 1938, 60, 309). In particular, the specific surface area values measured in the present invention have been calculated from the BET surface area plot obtained by measuring the quantity of nitrogen gas molecules adsorbed by a weighted amount of solid at different relative pressures ($P/P_0$) within the range 0.05-0.3, at 77 K. Precisely, the measurement of the adsorption of gas molecules was

carried out by means of a Micromeritics™ ASAP 2010 equipment having the characteristics as set out below. Namely, the gas used for adsorption measure was nitrogen gas. The analyzed sample was prepared by weighting dabigatran etexilate mesylate (approximately 0.5 g). The sample for analysis was degassed at 30 °C for 10 minutes and at 140 °C for 30 minutes. The determination of the adsorption of nitrogen was measured at 77 K and for twelve relative pressures sufficiently dispersed within the range of 0.05 to 0.3 [*i.e.* twelve absolute pressures in the range of 38 mmHg (5.1 kPa) to 228 mmHg (30.4 kPa) relative to a saturated pressure of 760.0 mmHg (101.3 kPa)].

The particle density value disclosed in the present invention for crystalline form I of dabigatran etexilate mesylate can be determined by means of helium pycnometry, which is a well-accepted theory known in the art for the experimental calculation of particle density values when the crystal structure is not available. In particular, the particle density of crystalline form I of dabigatran etexilate mesylate has been determined by using a helium gas pycnometer (AccuPyc 1330, Micromeritics™) at room temperature, and using helium to determine the volume of the sample by measuring the pressure change of helium in a calibrated volume. The analyzed sample was prepared by exactly weighting about 1.2 g of dabigatran etexilate mesylate and introducing it into the sample cell. Air and moisture were removed from the inside of the chamber by purging the sample cell. Measurement of the sample volume was carried out by filling the sample cell with helium to the required filling pressure (19.5 psig; *i.e.,* 134.4 kPa). Then, the gas was expanded in the expansion cell and the final pressure at equilibrium was recorded. The equilibration rate was 0.005 psig/min (*i.e.,* 34.47 Pa/min). The volume of the sample was directly obtained according to the following equation:

$$V_S = V_{SC} - \frac{V_{EC}}{\frac{P_r}{P_f} - 1}$$

where $V_S$ is the volume of the sample (cm$^3$); $V_{SC}$, the volume of the sample cell (cm$^3$); $V_{EC}$, the volume of the expansion cell (cm$^3$); $P_r$, the run filling pressure (psig); and $P_f$, the final pressure (psig). Concretely, the cell and expansion volumes were determined to be 12.1822 cm$^3$ and 8.3767 cm$^3$, respectively, after calibration using standard steel balls. Finally, the density of dabigatran etexilate mesylate was obtained by dividing the weight of sample by the measured volume.

The bulk density values disclosed in the present invention can be calculated by introducing the bulk dabigatran etexilate mesylate sample to be determined without compacting it into a 10 mL graduated cylinder and calculating the net weight of sample, *i.e.* the difference between the gross weight ($W_{gross}$) of the cylinder containing the sample and the tare weight ($W_{tare}$) of the empty cylinder, both expressed in grams. The bulk density was obtained by dividing the net weight of sample by the apparent volume ($V_0$) of the unsettled sample in mL, as read to the nearest graduated millimeter of the cylinder, according to the following equation:

$$\rho_{bulk} = \frac{W_{gross} - W_{tare}}{V_0}$$

The differential scanning calorimetry (DSC) measurements disclosed in the present invention were performed with a DSC823 equipment available from Mettler-Toledo at a heating rate of 10.0 °C/min from 30.0 °C to 210.0 °C and under a nitrogen flow of 50 mL/min. The samples were prepared by exactly weighting about 2 mg of dabigatran etexilate mesylate in a standard aluminum pan (40 μL volume), and closing it before the analysis. Results were collected and analyzed using STAR$^e$ software version 9.20 from Mettler-Toledo.

The term "about" when used in the present invention preceding a number and referred to it, is meant to designate any value which lies within the range defined by the number ±10% of its value, preferably a range defined by the number ±5%, more preferably a range defined by the number ±2%, still more preferably a range defined by the number ±1%. For example, "about 10" should be construed as meaning within the range of 9 to 11, preferably within the range of 9.5 to 10.5, more preferably within the range of 9.8 to 10.2, and still more preferably within the range of 9.9 to 10.1.

The following examples further illustrate the invention but, of course, should not be construed as in any way limiting its scope.

EXAMPLES

**General Experimental Conditions:**

The dabigatran etexilate (free base) used as a starting material in the examples below can be obtained by any

of the synthetic processes disclosed in the literature. The dabigatran etexilate was further purified by crystallization from ethyl acetate and dried. The HPLC purity of the resulting dabigatran etexilate that was used as a starting material for the preparation of its mesylate salt was higher than 99.5%.

**X-Ray Powder Diffraction (XRPD):**

The XRPD pattern was recorded on a Siemens D5000 diffractometer equipped with two symmetrically mounted vertical goniometers (Bragg-Brentano geometry) with horizontal sample stages, a X-ray tube, a high voltage generator (working at 45 kV and 35 mA) and standard scintillation detectors. Ni-filtered Cu-anode source was used and diffracted radiation was further monochromatized with a graphite crystal to avoid fluorescence effects [($\lambda(K_\alpha)$ = 1.54056 A]. The diffraction pattern was recorded including values of $2\theta$ that range from 2 to 50° with a sampling rate of 0.02° per second and a step time of 1 second per step. The powdered sample was pressed between two glass plates, forming a film. DIFFRAC Plus measurement software with EVA evaluation software (Bruker) was used to record the data and for a primary analysis of the diffraction pattern. The equipment was periodically calibrated using quartz and silicon.

**Particle Size Distribution (PSD):**

The particle size measurement for dabigatran etexilate mesylate was obtained using a Malvern Mastersizer S, light scattering based particle size analyzer equipped with a 2 milliwatt helium/neon laser and a Fourier Transform lens system. The sample was run using the 2.40 mm lens. The sample unit was a MS1 small volume sample dispersion unit stirred cell. The dispersant was Isopar™ G. The sample particle size distribution was assumed to follow a normal distribution.

Analysis model: polydisperse. Particle R.I. = (1.6140, 0.1000). Dispersant R.I. = 1.4200. Sample measurement time: 6 s. Background measurement time: 4 s. Stirrer speed: 2,500 r.p.m.

Samples for analysis were prepared by wetting a weighed amount of dabigatran etexilate mesylate (approximately 60 mg) with 20 mL of a 5% solution (w/v) of soybean lecithin in Isopar™ G, which was prepared by dissolving 2.5 g of soybean lecithin in 50 mL of Isopar™ G. This sample was sonicated in an ultrasound bath for 3 minutes and delivered dropwise to the previously background and corrected measuring cell filled with dispersant (Isopar™ G) until the obscuration reached the desired level (13-18%). Laser diffraction results were reported on a volume basis. Volume distributions were obtained for at least six measures. For characterization, the values of $D_{10}$, $D_{50}$ and $D_{90}$ (by volume) were specifically listed, each one being the mean of the six values available for each characterization parameter.

Alternatively, a Malvern Mastersizer 2000 equipment with a Hydro 2000S small volume sample dispersion unit stirred cell was also used for the particle size measurement of dabigatran etexilate mesylate. Measurement parameters were exactly the same as for the above method except for the background measurement time, which was set to 2 s. Samples for analysis were prepared by wetting a weighed amount of dabigatran etexilate mesylate (approximately 60 mg) with 20 mL of a 0.75% solution (w/v) of soybean lecithin in Isopar™ G, which was prepared by dissolving 1.5 g of soybean lecithin in 200 mL of Isopar™ G. Other parameters were not changed with respect to those disclosed in the above method for the Malvern Mastersizer S equipment. Methods were checked to be fully equivalent by performing a comparative analysis of a defined set of samples by using both particle size analysis methods.

**Specific Surface Area (SSA):**

The BET (Brunauer, Emmett and Teller) specific surface area for dabigatran etexilate mesylate was measured using a Micromeritics™ ASAP 2010 equipment (ASAP 2010 Software™). The sample for analysis was degassed at 30 °C for 10 minutes and at 140 °C for 30 minutes. The determination of the adsorption of $N_2$ at 77 K was measured for twelve relative pressures sufficiently dispersed within the range of 0.05 to 0.3.

**Particle density ($\rho_{particle}$):**

Particle density of dabigatran etexilate mesylate samples corresponding to crystalline form I was determined by using a helium gas pycnometer (AccuPyc 1330, Micromeritics™) at room temperature. The pycnometer used helium (99.995 % pure) to determine the volume of the sample, by measuring the pressure change of helium in a calibrated volume. The cell and expansion volumes were determined to be 12.1822 $cm^3$ and 8.3767 $cm^3$, respectively, after calibration by quintuplicate using standard steel balls. 1.2059 g of dabigatran etexilate mesylate were introduced in the sample cell. Working temperature was 26.1 °C. The cell was purged 5 times to clean up the sample and remove air and moisture from the inside of the chamber. Measurement of the sample volume was carried out by filling the sample cell with helium to the required filling pressure (19.5 psig; *i.e.,* 134.4 kPa). Then, the gas was expanded in the expansion cell and the final pressure at equilibrium was recorded. The equilibration rate was 0.005 psig/min (*i.e.,* 34.47 Pa/min).

The volume of the sample was directly obtained according to the following equation:

$$V_S = V_{SC} - \frac{V_{EC}}{\frac{P_r}{P_f} - 1}$$

where $V_S$ is the volume of the sample (cm$^3$); $V_{SC}$, the volume of the sample cell (cm$^3$); $V_{EC}$, the volume of the expansion cell (cm$^3$); $P_r$, the run filling pressure (psig); and $P_f$, the final pressure (psig). Finally, the density of dabigatran etexilate mesylate was obtained by dividing the weight of sample by the measured volume.

According to the above method, the particle density for dabigatran etexilate mesylate was experimentally found to be 1.308 g/cm$^3$.

**Bulk density ($\rho_{bulk}$):**

The bulk density was measured by introducing the bulk substance to be determined without compacting it into a 10 mL graduated cylinder and calculating the net weight of sample, *i.e.* the difference between the gross weight *($W_{gross}$)* of the cylinder containing the sample and the tare weight *($W_{tare}$)* of the empty cylinder, both expressed in grams. The bulk density was obtained by dividing the net weight of sample by the apparent volume ($V_0$) of the unsettled sample in mL, as read to the nearest graduated millimeter of the cylinder, according to the following equation:

$$\rho_{bulk} = \frac{W_{gross} - W_{tare}}{V_0}$$

**Differential Scanning Calorimetry (DSC):**

DSC measurements were performed with a DSC823 equipment available from Mettler-Toledo at a heating rate of 10.0 °C/min from 30.0 °C to 210.0 °C and under a nitrogen flow of 50 mL/min. The sample was prepared by exactly weighting about 2 mg of dabigatran etexilate mesylate in a standard aluminum pan (40 μL volume), and closing it before the analysis. Results were collected and analyzed using STAR$^e$ software version 9.20 from Mettler-Toledo.

**Reference Example 1: Precipitation of dabigatran etexilate mesylate from acetone**

610 g (972 mmol) of dabigatran etexilate were dissolved in 4.3 L of acetone at 40-45°C, under nitrogen atmosphere. The solution was filtered, and the filter was washed with 193 mL of acetone. A solution of 91.5 g (952 mmol, 0.98 molar equivalents) of methanesulfonic acid in 484 mL of acetone was added dropwise. Precipitation was observed during the addition. The addition funnel was rinsed with 131 mL of acetone. The resulting suspension was cooled to 20°C and filtered. The solid was washed with 948 mL of acetone and was dried at 60°C, to obtain 643.5 g of dabigatran etexilate mesylate. Yield: 93.4%. XRPD: Form I. Particle Size Distribution: $D_{10}$ 2.4 μm, $D_{50}$ 12.0 μm, $D_{90}$ 30.8 μm. Specific Surface Area: 3.06 m$^2$/g.

**Reference Example 2: Precipitation of dabigatran etexilate mesylate from a mixture of solvents comprising acetone (*i.e.,* DMSO / acetone)**

18.7 Kg (29.8 mol) of dabigatran etexilate were dissolved in 111.5 L of acetone at 40-45°C, under nitrogen atmosphere. The solution was filtered, and the filter was washed with 9.2 L of acetone. 22.3 L of dimethylsulfoxide were added to the solution. Then, a solution of 2.7 Kg (28.1 mol, 0.94 molar equivalents) of methanesulfonic acid in 4.7 L of acetone was added dropwise. The addition funnel was rinsed with 4.7 L of acetone. The solution was seeded with dabigatran etexilate mesylate (Form I). Precipitation was observed after the addition of seeds. The resulting suspension was cooled to between -5°C to 0°C and filtered. The solid was washed with 2 x 17.7 L of acetone and was dried at 60°C, to obtain 17.0 Kg of dabigatran etexilate mesylate. Yield: 83.6%. XRPD: Form I. Particle Size Distribution: $D_{10}$ 1.6 μm, $D_{50}$ 6.1 μm, $D_{90}$ 21.4 μm.

**Comparative Example 1: Milling of dabigatran etexilate mesylate**

The product obtained in Reference Example 2 was milled in a Micromill 160-Z stainless steel pin mill from Lleal S.A. operating at 14,000 rpm and was passed through a 500 μm sieve, to obtain 16.4 Kg of dabigatran etexilate mesylate. XRPD: Form I. Particle Size Distribution: $D_{10}$ 1.8 μm, $D_{50}$ 5.0 μm, $D_{90}$ 13.8 μm. Specific Surface Area: 5.66 $m^2/g$. Bulk density: 0.116 $g/cm^3$. Porosity: 0.91.

**Comparative Example 2: Micronizing of dabigatran etexilate mesylate**

75 g of the dabigatran etexilate mesylate obtained in Reference Example 1 were micronized in a Rina-jet turbomicronizer from Riera Nadeu S.A. using nitrogen at 20-25°C. Conditions: venturi at 6 bar, grinding chamber at 4 bar. XRPD: Form I. Particle Size Distribution: $D_{10}$ 0.6 μm, $D_{50}$ 2.6 μm, $D_{90}$ 5.5 μm. Specific Surface Area: 6.27 $m^2/g$. Bulk density: 0.193 $g/cm^3$. Porosity: 0.85.

**Example 1: Precipitation of dabigatran etexilate mesylate from tetrahydrofuran**

750 g (1.19 mol) of dabigatran etexilate were dissolved in 5.3 L of tetrahydrofuran at 15°C, under nitrogen atmosphere. The solution was filtered, and the filter was washed with 211 mL of tetrahydrofuran. The filtrate was heated to 25°C. Then, a solution of 112.5 g (1.17 mol, 0.98 molar equivalents) of methanesulfonic acid in 750 mL of tetrahydrofuran was added dropwise. Precipitation was observed during the addition. The addition funnel was rinsed with 425 mL of tetrahydrofuran. The resulting suspension was filtered. The solid was washed with 844 mL of tetrahydrofuran and subsequently with 2 x 836 mL of ethyl acetate, and was dried at 60°C to obtain 796 g of dabigatran etexilate mesylate. Yield: 94.0%. XRPD: Form I. Particle Size Distribution: $D_{10}$ 3.9 μm, $D_{50}$ 15.2 μm, $D_{90}$ 32.9 μm.

**Example 2: Precipitation of dabigatran etexilate mesylate from tetrahydrofuran**

8.88 Kg (14.15 mol) of dabigatran etexilate were dissolved in 62.2 L of tetrahydrofuran at 20°C, under nitrogen atmosphere. The solution was filtered, and the filter was washed with 5.6 L of tetrahydrofuran. The filtrate was heated to 25°C. Then, a solution of 1.33 Kg (13.84 mol, 0.98 molar equivalents) of methanesulfonic acid in 8.9 L of tetrahydrofuran was added dropwise. Precipitation was observed during the addition. The resulting suspension was filtered. The solid was washed with 8.9 L of tetrahydrofuran and subsequently with 2 x 8.9 L of ethyl acetate, and was dried at 60°C to obtain 9.60 Kg of dabigatran etexilate mesylate. Yield: 95.8%. XRPD: Form I. Particle Size Distribution: $D_{10}$ 0.3 μm, $D_{50}$ 10.6 μm, $D_{90}$ 30.1 μm.

**Example 3: Milling of dabigatran etexilate mesylate**

The product obtained in Example 2 was milled in a Micromill 160-Z stainless steel pin mill from Lleal S.A. operating at 14,000 rpm and was passed through a 500 μm sieve, to obtain 8.3 Kg of dabigatran etexilate mesylate. XRPD: Form I. DSC: No endotherm detected between 185.0 °C and 195.0 °C. Particle Size Distribution: $D_{10}$ 1.4 μm, $D_{50}$ 7.0 μm, $D_{90}$ 19.7 μm. Specific Surface Area: 10.48 $m^2/g$. Bulk density: 0.188 $g/cm^3$. Porosity: 0.86.

**Example 4: Micronizing of dabigatran etexilate mesylate**

75 g of the dabigatran etexilate mesylate obtained in Example 1 were micronized in a Rina-jet turbomicronizer from Riera Nadeu S.A. using nitrogen at 20-25°C. Conditions: venturi at 6 bar, grinding chamber at 4 bar. XRPD: Form I. DSC: No endotherm detected between 185.0 °C and 195.0 °C. Particle Size Distribution: $D_{10}$ 0.6 μm, $D_{50}$ 2.8 μm, $D_{90}$ 6.6 μm. Specific Surface Area: 8.74 $m^2/g$. Bulk density: 0.181 $g/cm^3$. Porosity: 0.86.

**Example 5: Micronizing of dabigatran etexilate mesylate**

50 g of the dabigatran etexilate mesylate obtained in Example 2 were micronized in a Rina-jet turbomicronizer from Riera Nadeu S.A. using nitrogen at 20-25°C. Conditions: venturi at 6 bar, grinding chamber at 4 bar. XRPD: Form I. DSC: No endotherm detected between 185.0 °C and 195.0 °C. Particle Size Distribution: $D_{10}$ 0.2 μm, $D_{50}$ 2.4 μm, $D_{90}$ 5.0 μm. Specific Surface Area: 9.23 $m^2/g$. Bulk density: 0.197 $g/cm^3$. Porosity: 0.85.

**Example 6: Micronizing of dabigatran etexilate mesylate**

15 g of the dabigatran etexilate mesylate obtained in Example 5 were micronized in a Rina-jet turbomicronizer

from Riera Nadeu S.A. using nitrogen at 20-25°C. Conditions: venturi at 6 bar, grinding chamber at 4 bar. XRPD: Form I. DSC: No endotherm detected between 185.0 °C and 195.0 °C. Particle Size Distribution: $D_{10}$ 0.2 μm, $D_{50}$ 2.0 μm, $D_{90}$ 4.3 μm. Specific Surface Area: 9.15 $m^2$/g. Bulk density: 0.171 g/$cm^3$. Porosity: 0.87.

**Example 7: Data comparison between batches**

Comparison of particle size distribution, specific surface area (SSA), bulk density and porosity values between different batches of mechanically comminuted dabigatran etexilate mesylate is summarized in table 1, below.

**Table 1**

| Example | Solvent for starting dabigatran etexilate mesylate | Comminution process | $D_{90}$ (μm) | $D_{50}$ (μm) | $D_{10}$ (μm) | SSA ($m^2$/g) | Bulk density (g/$cm^3$) | Porosity |
|---|---|---|---|---|---|---|---|---|
| Comp. 1 | DMSO/acetone | Milling | 13.8 | 5.0 | 1.8 | 5.66 | 0.116 | 0.91 |
| Comp. 2 | Acetone | Micronizing | 5.5 | 2.6 | 0.6 | 6.27 | 0.193 | 0.85 |
| 3 | THF | Milling | 19.7 | 7.0 | 1.4 | 10.48 | 0.188 | 0.86 |
| 4 | THF | Micronizing | 6.6 | 2.8 | 0.6 | 8.74 | 0.181 | 0.86 |
| 5 | THF | Micronizing | 5.0 | 2.4 | 0.2 | 9.23 | 0.197 | 0.85 |
| 6 | THF | Micronizing | 4.3 | 2.0 | 0.2 | 9.15 | 0.171 | 0.87 |

## Claims

1. Dabigatran etexilate mesylate, **characterized by** a specific surface area in the range of from about 7.0 $m^2$/g to about 20 $m^2$/g, preferably from about 7.5 $m^2$/g to about 15 $m^2$/g, and even more preferably from about 8.0 $m^2$/g to about 13 $m^2$/g.

2. Dabigatran etexilate mesylate according to claim 1, **characterized in that** it is mechanically comminuted.

3. Dabigatran etexilate mesylate according to claim 2, obtained by a mechanical comminution method selected from the group consisting of cutting, chipping, crushing, milling, micronizing and triturating.

4. Dabigatran etexilate mesylate according to any one of claims 1 to 3, having a $D_{90}$ of less than about 25 μm, preferably less than about 22 μm, and even more preferred less than about 20 μm.

5. Dabigatran etexilate mesylate according to any one of claims 1 to 4, having a $D_{50}$ of less than about 15 μm, preferably less than about 12 μm, and even more preferred less than about 10 μm.

6. Dabigatran etexilate mesylate according to any one of claims 1 to 5, having a $D_{10}$ of less than about 5 μm, preferably less than about 3 μm, and even more preferred less than about 2 μm.

7. Dabigatran etexilate mesylate according to any one of claims 1 to 6, wherein the dabigatran etexilate mesylate is in crystalline form I.

8. Dabigatran etexilate mesylate according to claim 7, having a measured experimental particle density of from about 1.200 g/$cm^3$ to about 1.400 g/$cm^3$, preferably from about 1.250 g/$cm^3$ to about 1.350 g/$cm^3$, and even more preferred of from about 1.300 g/$cm^3$ to about 1.320 g/$cm^3$, as measured by helium pycnometry.

9. Dabigatran etexilate mesylate according to claim 7, which does not show any detectable endothermic peak between 185.0°C and 195.0°C as measured by differential scanning calorimetry at a heating rate of 10 °C/min.

10. Dabigatran etexilate mesylate according to any one of claims 7 to 9, having a bulk density of at least about 0.150 g/$cm^3$.

11. Dabigatran etexilate mesylate according to any one of claims 7 to 10, having a porosity not higher than about 0.90.

**12.** Dabigatran etexilate mesylate according to any one of claims 1 to 11, wherein the dabigatran etexilate mesylate is obtained by a process comprising:

i) providing a solution of dabigatran etexilate in an ether solvent;
ii) combining the solution of step (i) with methanesulfonic acid;
iii) optionally seeding with dabigatran etexilate mesylate in crystalline form I;
iv) removing the solvent to obtain dabigatran etexilate mesylate; and
v) optionally mechanical comminution.

**13.** Dabigatran etexilate mesylate according to claim 12, wherein the ether solvent is selected from a group comprising 1,2-dimethoxyethane, 1,4-dioxane, tetrahydrofuran, 2-methyltetrahydrofuran, anisole, tert-butyl methyl ether, diethyl ether, diisopropyl ether, diphenyl ether, cyclopentyl methyl ether, 1,1-diethoxypropane, 1,1-dimethoxypropane, 2,2-dimethoxypropane and mixtures thereof, preferably from a group comprising tetrahydrofuran, 2-methyltetrahydrofuran and mixtures thereof, more preferably comprising tetrahydrofuran.

**14.** A process for preparing dabigatran etexilate mesylate according to any one of claims 1 to 13, said process comprising:

i) providing dabigatran etexilate mesylate; and
ii) reducing the particle size of the dabigatran etexilate mesylate of step (i) with a method selected from the group consisting of cutting, chipping, grinding, crushing, milling, micronizing and triturating.

**15.** The process of claim 14 comprising:

a) milling dabigatran etexilate mesylate;
b) sieving the milled crystals through a screen having a diameter of 1000 μm or less;
c) separating the sieved crystals; and
d) optionally repeating steps (a) to (c).

**16.** The process of claim 15, wherein said milling comprises air-jet milling or pin milling.

**17.** The process of any one of claims 14 to 16, wherein the dabigatran etexilate mesylate of step (i) is obtained by a process comprising:

a) providing a solution of dabigatran etexilate in an ether solvent;
b) combining the solution of step (a) with methanesulfonic acid;
c) optionally seeding with dabigatran etexilate mesylate in crystalline form I; and
d) removing the solvent to obtain dabigatran etexilate mesylate.

**18.** The process of claim 17, wherein the ether solvent is selected from a group comprising 1,2-dimethoxyethane, 1,4-dioxane, tetrahydrofuran, 2-methyltetrahydrofuran, anisole, tert-butyl methyl ether, diethyl ether, diisopropyl ether, diphenyl ether, cyclopentyl methyl ether, 1,1-diethoxypropane, 1,1-dimethoxypropane, 2,2-dimethoxypropane and mixtures thereof, preferably from a group comprising tetrahydrofuran, 2-methyltetrahydrofuran and mixtures thereof, more preferably comprising tetrahydrofuran.

**19.** A pharmaceutical composition comprising dabigatran etexilate mesylate according to any of claims 1 to 13 and one or more pharmaceutically acceptable excipients.

**20.** A pharmaceutical composition comprising dabigatran etexilate mesylate and one or more pharmaceutically acceptable excipients, wherein said dabigatran etexilate mesylate is formed from dabigatran etexilate mesylate according to any of claims 1 to 13.

**21.** A process for producing a pharmaceutical composition wherein dabigatran etexilate mesylate according to any one of claims 1 to 13 is admixed with one or more pharmaceutically acceptable carriers, diluents or excipients.

**22.** The pharmaceutical composition of claim 19 or 20 or as defined in claim 21, selected from the group consisting of an oral suspension, coated tablets, non-coated tablets, orodispersible tablets, pellets, pills, granules, capsules, and mini-tablets in capsules.

**23.** A method for the prophylaxis and/or treatment of thromboembolic diseases in a patient comprising administering to said patient a therapeutically effective dose of dabigatran etexilate mesylate according to any one of claims 1 to 13.

**24.** A method for the prophylaxis and/or treatment of thromboembolic diseases in a patient comprising administering to said patient a pharmaceutical composition according to any one of claims 19, 20 and 22 or as defined in claim 21.

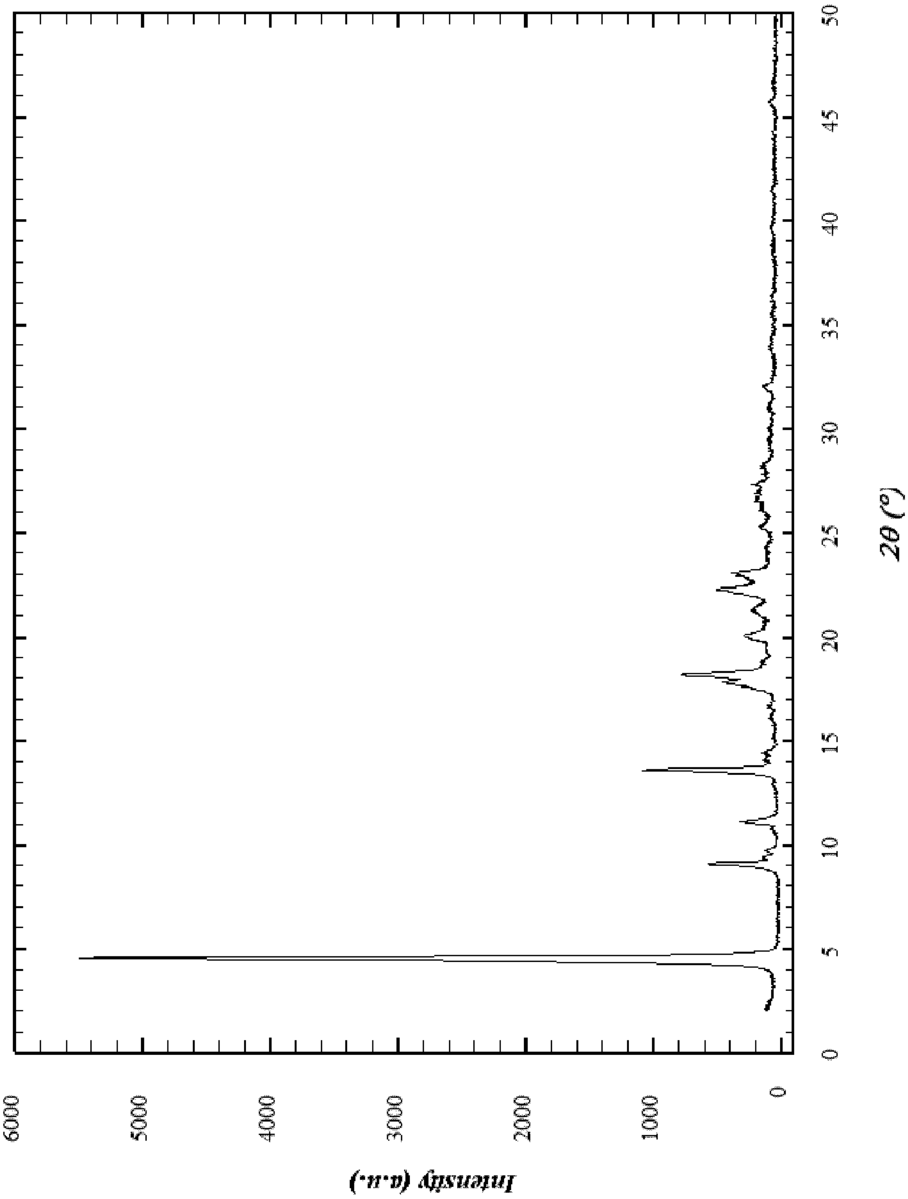


**Figure 1. XRPD of Form I of dabigatran etexilate mesylate**

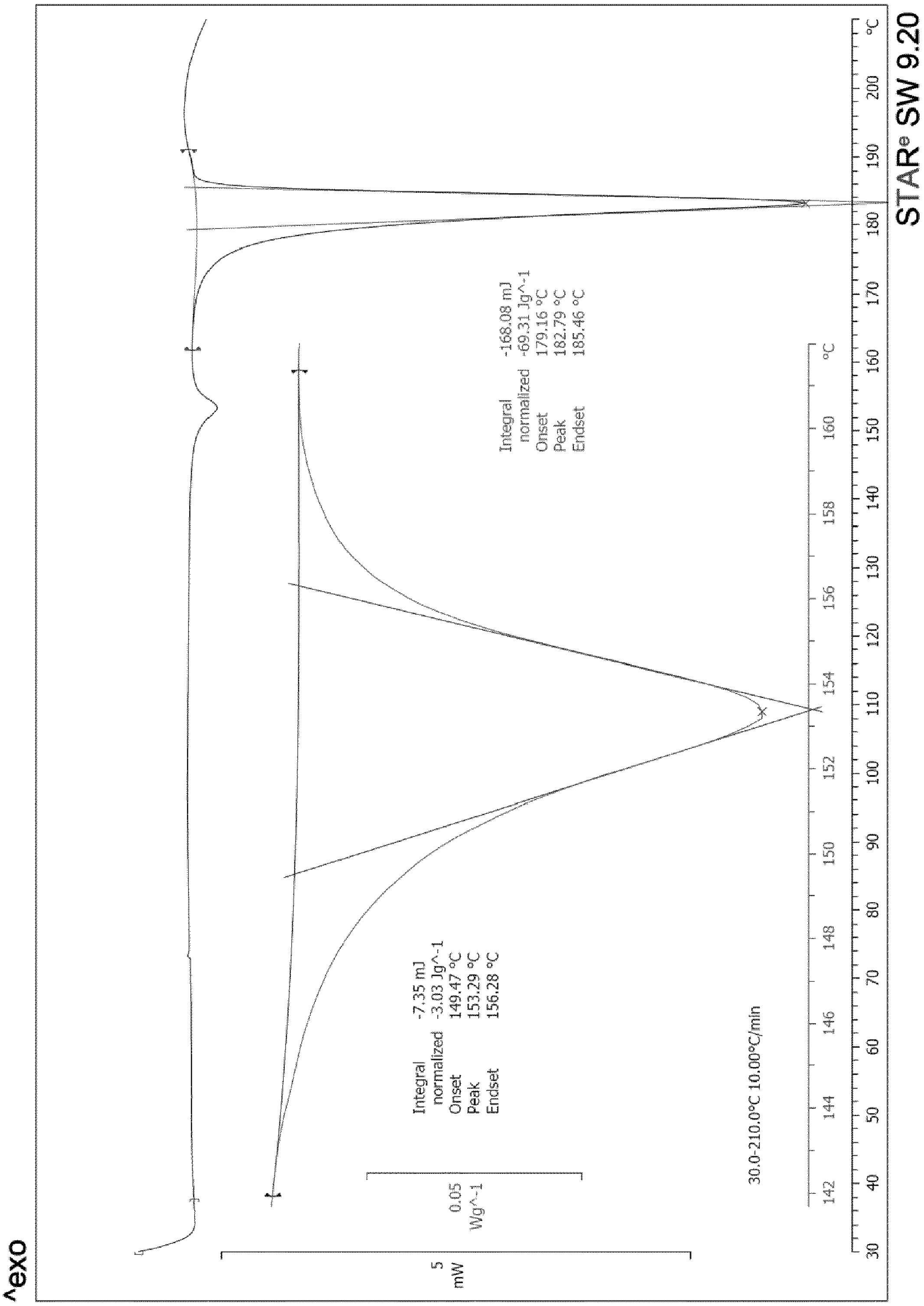


**Figure 2. DSC of Form I of dabigatran etexilate mesylate, substantially free of Form II**

Europäisches Patentamt
European Patent Office
Office européen des brevets

# EUROPEAN SEARCH REPORT

Application Number
EP 13 17 9693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/111163 A2 (CADILA HEALTHCARE LTD [IN]) 1 August 2013 (2013-08-01) * page 14, line 10 - page 15, line 29; claims 33-38; figure 5; example 8 * ----- | 1-24 | INV. C07D401/12 A61K31/4439 A61P7/02 |
| X,D | WO 2012/001156 A2 (KRKA TOVARNA ZDRAVIL D D NOVO MESTO [SI]; KROSELJ VESNA [SI]) 5 January 2012 (2012-01-05) * page 25; example C2 * * page 12, paragraph 3 - paragraph 4 * ----- | 1-24 | |
| X,D | WO 2012/077136 A2 (MSN LAB LTD [IN]; SATYANARAYANA REDDY MANNE [IN]; NAGARAJU CHAKILAM [I) 14 June 2012 (2012-06-14) * page 25, line 15 - line 19; example 12 * * page 14, line 30 - page 15, line 9; figures 4,5 * ----- | 1-24 | |
| X,D | WO 2012/027543 A1 (TEVA PHARMA [US]; PLUS CHEMICALS SA [CH]; LEKSIC EDISLAV [HR]; DOGAN J) 1 March 2012 (2012-03-01) * examples 1,22b * ----- | 1-24 | TECHNICAL FIELDS SEARCHED (IPC) |
| E | WO 2013/150545 A2 (MSN LAB LTD [IN]; THIRUMALAI RAJAN SRINIVASAN [IN]; ESWARAIAH SAJJA [I) 10 October 2013 (2013-10-10) * page 23, line 20 - line 25; claims 37-39; examples 14,15 * ----- | 1,7,8, 11-14, 17-24 | C07D A61K A61P |

The present search report has been drawn up for all claims

1

| Place of search | Date of completion of the search | Examiner |
|---|---|---|
| Munich | 18 November 2013 | Papathoma, Sofia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 9693

This annex lists the patent family members relating to the patent documents cited in the above-mentioned European search report. The members are as contained in the European Patent Office EDP file on
The European Patent Office is in no way liable for these particulars which are merely given for the purpose of information.

18-11-2013

| Patent document cited in search report | Publication date | Patent family member(s) | Publication date |
|---|---|---|---|
| WO 2013111163 A2 | 01-08-2013 | NONE | |
| WO 2012001156 A2 | 05-01-2012 | EA 201291421 A1 | 30-05-2013 |
| | | EP 2588090 A2 | 08-05-2013 |
| | | US 2013177652 A1 | 11-07-2013 |
| | | WO 2012001156 A2 | 05-01-2012 |
| WO 2012077136 A2 | 14-06-2012 | EP 2649060 A2 | 16-10-2013 |
| | | WO 2012077136 A2 | 14-06-2012 |
| WO 2012027543 A1 | 01-03-2012 | NONE | |
| WO 2013150545 A2 | 10-10-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1870100 B1
- WO 2011110876A1 A
- US 7202368 B2
- US 7932273 B2
- US 8354543 B2
- US 8394962 B2
- US 8399678 B2
- WO 2012077136A2 A
- WO 2012152855 A1
- WO 2013024384 A1
- US 7880016 B2
- WO 2012152855A1 A
- WO 2012027543A1 A
- WO 2012001156A2 A
- WO 2011110478A1 A


### Non-patent literature cited in the description


- *Eur. J. Pharm. Sci.,* 2006, vol. 27, 19-26
- *Pharm. Dev. Technol.,* 2004, vol. 9, 1-13
- **S. BRUNAUER ; P.H. EMMETT ; E. TELLER.** *J. Am. Chem. Soc.,* 1938, vol. 60, 309